Europäisches Patentamt

European Patent Office

Office européen des brevets

(11) Publication number: **0 445 414 A2**

# EUROPEAN PATENT APPLICATION

(21) Application number: **90124948.2**

(22) Date of filing: **20.12.90**

(51) Int. Cl.⁵: **C07K 3/26, C12N 1/20, C07K 3/28, C12P 21/00, //A23J1/00,(C12P21/00, C12R1:01)**

(30) Priority: **05.03.90 US 488132**

(43) Date of publication of application:
**11.09.91 Bulletin 91/37**

(84) Designated Contracting States:
**AT BE CH DE DK ES FR GB GR IT LI LU NL SE**

(71) Applicant: **MICROLIFE TECHNICS, INC.**
**1833 57th Street**
**Sarasota, Florida 34230(US)**

(72) Inventor: **Vandenbergh, Peter A.**
**4414 Meadowcreek Circle**
**Sarasota, Florida 33583(US)**
Inventor: **Kunka, Blair S.**
**1235D Carlton Arms Circle**
**Bradenton, Florida 34208(US)**
Inventor: **Henderson, James T.**
**6719 5th Street, W.**
**Bradenton, Florida 34207(US)**

(74) Representative: **Patentanwälte Grünecker, Kinkeldey, Stockmair & Partner**
**Maximilianstrasse 58**
**W-8000 München 22(DE)**

(54) **Ultrafiltration method for purification of a pediococcal bacteriocin.**

(57) Purification of a bacteriocin from a Pediococcus by ultrafiltration of supernatant of a culture of the Pediococcus and having a molecular weight of between about 4,000 and 5,000 daltons is described. The bacteriocin is useful in foods to inhibit bacterial growth.

## BACKGROUND OF THE INVENTION

### (1) Field of the Invention

The present invention relates to a method for purifying a pediococcal bacteriocin by ultrafiltration. In particular, the present invention relates to a method wherein the purified bacteriocin retains its activity after purification.

### (2) Prior Art

Proteins can be isolated using a variety of procedures that include precipitation with inorganic salts, organic solvents, ion exchanges, molecular sieve chromatography and ultrafiltration. Every protein reacts differently to each of the above methods and the purification achieved and the amount and activity of the protein recovered varies greatly. Ultrafiltration which was developed in the 1960's has been successfully utilized to purify a variety of proteins. Different types of protein ultrafiltration membranes and systems have been developed.

Tangential flow filtration is a separation technique that works by sweeping larger retained molecules across the surface of the membrane. The process can be used to purify and collect material passing through the membrane (filtrate) or material retained by the membrane (retentate). Molecules smaller than the pore size or molecular weight cutoff (MWCO) are able to pass through the membrane and are thus separated from higher molecular weight molecules.

Molecular ultrafiltration is a form of barrier filtration wherein molecules having a size between about $10^{-3}$ and $10^{-1}$ microns (10 to 1000 angstroms) are separated. Molecules are selectively separated by molecular weight cutoff. The separation is accomplished at pressures between about 10 and 100 psi. The filters are generally hollow fiber membrane, plate and frame and spiral tubular. Such apparatus are well known to those skilled in the art.

Chemical and Engineering News 32-54 (April 10, 1989) discuss protein folding and the affects on activity. A change in shape (configuration) of the protein changes the biological activity. The shape of a protein molecule can be changed when it is purified. This result was found when purifying pediococcal bacteriocins from a growth medium. Attempts to purify these bacteriocins resulted in a loss of activity, even though the amount of protein isolated was increased as a result of the purification step. It was then realized that the purification step was changing the structure of the pediococcal bacteriocin in a manner which reduces the activity.

Another problem has been to purify pediococcal bacteriocins so that they do not contribute a flavor to foods in which they are incorporated. This requires a high activity per unit volume of the bacteriocin. This purification is essential if these bacteriocins are to be used in foods.

## OBJECTS

It is therefore an object of the present invention to provide a method wherein a pediococcal bacteriocin is produced with enhanced activity per unit volume upon purification. Further, it is an cbject to provide a bacteriocin which does not impart any flavor to foods at bacteriocidally effective levels. Further it is an object of the present invention to provide a method for producing the bacteriocin which is relatively simple and economical to perform. These and other objects will become increasingly apparent by reference to the following description.

## GENERAL DESCRIPTION

The present invention relates to a method for producing a bacteriocin which comprises: culturing a Pediococcus in a liquid and solid growth medium to produce the bacteriocin in the liquid; removing the solids from the liquid of the growth medium; and removing impurities present in the liquid by ultrafiltration to produce a retentate containing the bacteriocin in the liquid, wherein the retentate containing the bacteriocin has a higher activity per unit volume than the liquid growth medium containing the bacteriocin.

Further the present invention relates to a bacteriocin for use in foods which has been produced by a method comprising culturing a Pediococcus in a mixed liquid and solid growth medium to produce the bacteriocin in the liquid growth medium and removing impurities present in the liquid by ultrafiltration to produce a retentate containing the bacteriocin in the liquid, wherein the retentate containing the bacteriocin has a higher activity per unit volume than the liquid growth medium containing the bacteriocin and wherein the bacteriocin has a molecular weight between about 4,000 and 5,000 daltons, is stable in boiling water and is tasteless in foods at levels which inhibit the growth of bacteria present in the food.

It has been found that even though the preferred bacteriocin has a molecular weight of between about 4,000 to 5,000 daltons, it separates as if it was more than three times this size (about 16,500 daltons). Thus the ultrafiltration is conducted so that the retentate contains the bacteriocin even though it could be expected to pass through the ultrafiltration filter.

The preferred pediococcal bacteriocin is produced by a strain of Pediococcus acidilactici, preferably NRRL-B-18050. This strain is described in U.S. Patent No. 4,883,673 and has been deposited under the Budapest Treaty with the Northern Regional Research Laboratory in Peoria, Illinois.

The preferred pediococcal bacteriocin produced by Pediococcus acidilactici NRRL-B-18050 has a molecular weight of between about 4,000 and 5,000 daltons. The purified bacteriocin is stable in boiling water and retains its activity. Other pediococcal bacteriocins can be isolated by the method of the present invention.

The ultrafiltration filters separate the bacteriocin in the retentate so that it is not impaired by the filtration. Preferably the filter used has a cutoff between about 4,000 and 16,000 daltons so that the retentate contains the bacteriocin.

The growth medium for the Pediococcus includes a hydrolyzed protein, amino acids, a sugar and mineral supplements which stimulate growth. Such media are well known to those skilled in the art and are formulated to maximize production of the bacteriocin in the liquid of the growth medium. The ultrafiltrate can be treated with a precipitating agent, such as ammonia sulfate which essentially "salts out" the bacteriocin and then separated from the liquid. The ultrafiltrate can be dialyzed in a buffer through dializer to remove low molecular weight compounds (less than about 10 K daltons).

The ultrafiltrate containing the bacteriocin is preferably dried to a powder for incorporation into a food or for other uses. This prevents unwanted liquid from being introduced into the food and provides ease of shipping. Preferably the drying is by lyophilization, spray drying, drum drying, tray drying or thin film evaporation or any method where heat is applied below the level of heat inactivation of the bacteriocin. The ultrafiltration can be frozen as well and the activity is preserved.

The amount of lyophilized powder used in the food is up to about ten percent (10%) by weight of the food. Preferably the amount is between about 0.1 and 10 percent by weight of the food.

The resulting ultrafiltrate has an AU of at least about 100 AU per milliliter. Usually the AU is between about 100 and 16,000 per ml. One AU of bacteriocin defined as 5 microliters of the highest dilution of culture supernatent yielding a defined zone of inhibition with a layer of a Gram-positive bacteria on an agar plate (P. pentosaceus FBB-63 (formerly known as Pediococcus cerevisiae FBB-63). U.S. Patent No. 4,883,673 and application Serial No. 148,044 assigned to a common assignee, describe the use of the bacteriocin in foods. The bacteriocin in the culture or growth medium was filter sterilized at 0.22 micron to remove bacteria and other cell contaminants. There is no separation of various molecules in solution.

SPECIFIC DESCRIPTION

Strains: The bacterial isolate Pediococcus acidilactici NRRL-B-18050 was stored in liquid nitrogen and routinely cultured at 35° C on MRS Agar (Difco, Detroit, MI).

Medium: The Pediococcus acidilactici NRRL-B-18050 was grown in different quantities of the following medium. The medium contained: 4% corn steep (minerals and peptides), 5% glucose (sugar), 3% yeast extract (proteins and vitamins) and 1% HycaseTM (Sheffield, Norwich, New York) (an acid hydrolyzed casein protein which liberates amino acids and proteins). The medium was adjusted to pH 7.0 and sterilized.

Bacteriocin Assay: Production of bacteriocin was assayed as previously described by Gonzalez and Kunka (Applied and Environmental Microbiology 53: 2534-2538, 1987). Samples were filter sterilized using an 0.22 um (pore size) (Millipore Corp., Bedford, MA) filter. MRS Agar plates were overlaid with soft agar (0.8%) seeded with indicator cells. The filter sterilized samples were diluted in sterile dilution water and spotted (5 ul) onto the surface of the overlays.

Protein Assays: The micro-biuret protein determinative method of Koch and Putnam (Analytical Biochemistry 44: 239-245, 1971) was utilized. The protein standard was bovine serum albumin.

Examples 1 to 3 show various types of ultrafiltration. Example 4 shows the use of the bacteriocin. Example 5 shows the use of ammonium bicarbonate as a buffer for dialysis. The resulting lyophilized powder had a better taste when incorporated into food.

Example 1

Comparison of Ultrafiltration and Ammonium Sulfate precipitation on the concentration of PA-1.

Pediococcus acidilactici NRRL-B-18050 was grown for 18 hours at 35°C in 2 liters of the previously described medium. After 18 hours the broth culture was centrifuged at 8,000 x g for 20 minutes at 4°C to remove cells and other cellular impurities. The supernatant (1.0 1) was retained and subjugated to different types of purification. Ultrafiltration was accomplished using an Amicon™ Model 8200 Ultrafiltration cell (Division of W. R. Grace Co., Danver, MA). The supernatant was filtered through an ultrafiltration membrane YM10 (10,000 daltons molecular weight cutoff (MWCO)). The retentate was collected and assayed for PA-1 activity (Table 1). The remaining supernatant (1.0 1) was then subjected to ammonium sulfate precipitation (50% wt/vol), dialyzed against 0.01 M Tris-maleate pH 6.0 and assayed for PA-1 activity (Table 1).

## TABLE 1

| FRACTION | VOL(ML) | UNITS AU/ML | TOTAL UNITS | PROTEIN (MG/ML) | RECOVERY* % (Units) |
|---|---|---|---|---|---|
| ULTRAFILTRATION | | | | | |
| Supernatant | 1000 | 1,600 | $1.6 \times 10^6$ | 6.0 | 100 |
| 10,000 retentate | 40 | 32,000 | $1.3 \times 10^6$ | 45.0 | 81 |
| AMMONIUM SULFATE PRECIPITATION | | | | | |
| Dialyzed 50% ppt | 7 | 51,200 | $3.6 \times 10^5$ | 3.0 | 23 |

*Based upon Total Units in the crude

Example 1 shows that the 10,000 MWCO ultrafiltration produces a large recovery of bacteriocin which has a high activity in the retentate. This is true even though the protein has a size of between about 4,000 and 5,000 daltons. This indicates that the molecular weight of the bacteriocin is larger for ultrafiltration possibly because of aggregation of the molecules.

Example 2

Filtration of PA-1 using tangential flow ultrafiltration.

Pediococcus acidilactici NRRL-B-18050 was grown for 18 hours at 35°C in 1 liter of the previously described medium of Example 1. After 18 hours the broth culture was centrifuged at 8,000 x g for 20 minutes at 4°C. The supernatant (1.0 1) was retained and subjected to different types of purification. Ultrafiltration was accomplished using a Minitan™ tangential flow system (Millipore Corp., Bedford, MA). Two membrane plate sets were utilized, 100,000 (MWCO) and 10,000 (MWCO). The results are set forth in Table 2.

TABLE 2

| FRACTION | VOL(ML) | UNITS AU/ML | TOTAL UNITS | PROTEIN (MG/ML) | RECOVERY* % (Units) |
|---|---|---|---|---|---|
| TANGENTAL FLOW ULTRAFILTRATION | | | | | |
| 100,000 permeate | 900 | 800 | 720,000 | 18.5 | 100 |
| 10,000 RETENTATE | 350 | 1,600 | 560,000 | 30.0 | 78 |
| 10,000 MWCO permeate | 550 | 0 | 0 | 12.0 | 0 |

*Based upon Total Units in 100,000 MWCO permeate

This Example shows a large amount of the bacteriocin is recovered. Again the bacteriocin remained in the retentate even though its size was less than the MWCO.

Example 3

Filtration of PA-1 using spiral cartridge ultrafiltration.

Pediococcus acidilactici NRRL-B-18050 was grown for 18 hours at 35°C in 300 gallons of the previously described medium. After 18 hours the broth culture was centrifuged, the supernatant was retained and subjected to different types of purification. Ultrafiltration was accomplished using the PUF-15TM pilot system (Millipore Corp., Bedford, MA) which provides tangential flow. The supernatant was filtered through a 100,000 (MWCO) polysulfone spiral cartridge. The 100,000 (MWCO) permeate was then filtered through a 10,000 (MWCO) cellulosic spiral cartridge. The results are observed in Table 3.

TABLE 3

| FRACTION | VOL(ML) | UNITS AU/ML | TOTAL UNITS | PROTEIN (MG/ML) | RECOVERY* % (Units) |
|---|---|---|---|---|---|
| 100,000 permeate | 27.6 | 3200 | 88,320 | 33.5 | 100 |
| 10,000 retentate | 13.7 | 6400 | 87,680 | 30.5 | 99% |

*Based upon 100,000 permeate

This example shows the best recovery of the protein. The bacteriocin did not pass through the 10,000 MWCO even though it was smaller in size.

Example 4

Use of a lyophilized bacteriocin PA-1 produced by P. acidilactici B-NRRL-18050 that had been concentrated by ultrafiltration.

The bacteriocin PA-1 was produced by fermentation in the previously described medium. The bacteriocin was then concentrated by the method described in Example 3. The material was then lyophilized. The lyophilized material had an activity of 16,000 AU/g.

Commercially sterile canned chicken was inoculated with Listeria monocytogenes at a rate of $5 \times 10^3$ cfu/g of meat which is a heavy overload of this bacteria. The bacteriocin PA-1 was then added to the chicken at a rate of 1600 AU/g meat. The chicken was then stored at 7°C and aliquots were then sampled for the growth of L. monocytogenes using standard plate count procedures followed by plating on McBride's Agar (Difco, Detroit, MI). The results are depicted in Table 4.

## TABLE 4

| Incubation of chicken meat at 7°C (days) | Growth of L. monocytogenes cfu/g | |
|---|---|---|
| | Control (No PA-1) | Experimental (with PA-1) |
| 0 | $1.0 \times 10^4$ | $4.0 \times 10^3$ |
| 4 | $1.3 \times 10^7$ | $3.0 \times 10^3$ |
| 6 | $1.1 \times 10^9$ | $2.0 \times 10^3$ |

The results show that the lyophilized bacteriocin from the bacteriocin protected the canned chicken and therefore is useful in the extension of shelf life of the processed food. It was determined that the bacteriocin has no taste in the food.

Example 5

Use of ammonium bicarbonate in the diafiltration and concentration procedures to replace undesirable low molecular weight components.

Pediococcus acidilactici was grown for 18 hours at 35°C in 1 liter of the previously described medium of Example 1. After 18 hours the broth culture was centrifuged at 8,000 x g for 20 minutes at 4°C. The supernatant (1 liter) was filtered (.45 micron) and then ultrafiltered using the tangential flow system with 100,000 MWCO plates. The permeate was concentrated using 10,000 MWCO plates until 20% of the original volume remained (200 ml) as the retentate. Dialysis was performed by adding an equal volume of ammonium bicarbonate buffer (0.1 M, pH 7.8) to the permeate and then reducing the volume by one-half. This operation was done three times to give a final buffer concentration of 0.0875 M ammonium bicarbonate.

Diafiltered medium was lyophilized and the powder was tested by organoleptic evaluation. The bitter flavor present in cell-free broth culture and in concentrates thereof was absent in the ammonium bicarbonate treated sample because the ammonium bicarbonate is volatilized during lyophilization.

Other methods of purifying the bacteriocin were tried with very limited success. Included were: (1) ethanol precipitation and separation; (2) dialysis, centrifugal filtration, and ultrafiltration at 1,000 MWCO rather than 10,000 MWCO. None of these methods provided more than about four times increase in the activity in AU per ml. Thus the pediococcal bacteriocin requires separation by the method of the present invention to produce a high activity.

It is intended that the foregoing description be only illustrative of the present invention and that the present invention be limited only by the hereinafter appended claims.

**Claims**

1.  A method for producing a bacteriocin which comprises:

    (a) culturing a Pediococcus in a liquid and solid growth medium to produce the bacteriocin in the liquid;

    (b) removing the solids from the liquid of the growth medium; and

    (c) removing impurities present in the liquid by ultrafiltration to produce a retentate containing the bacteriocin in the liquid, wherein the retentate containing the bacteriocin has a higher activity per unit volume than the liquid growth medium containing the bacteriocin.

2. The method of Claim 1 wherein the bacteriocin is dried to a dry powder.

3. The method of Claim 1 wherein the growth medium included a protein, an amino acid, a sugar and mineral supplements which stimulate the production of the bacteriocin.

4. The method of Claim 1 wherein the liquid of the retentate is treated with a precipitating agent to precipitate the bacteriocin which is separated from the liquid.

5. The method of Claim 4 wherein the precipitation agent is ammonium sulfate.

6. The method of Claim 4 wherein the precipitated bacteriocin is dried to a dry powder.

7. The method of Claim 1 wherein the liquid of the retentate is treated with a precipitating agent to precipitate the bacteriocin which is separated from the liquid and then the bacteriocin is resuspended in a buffer and dialyzed to remove impurities in the bacteriocin.

8. The method of Claim 1 wherein the Pediococcus is Pediococcus acidilactici.

9. The method of Claim 1 wherein the Pediococcus is Pediococcus acidilactici NRRL-B-18050.

10. A bacteriocin for use in foods which has been produced by a method comprising:
    (a) culturing a Pediococcus in a mixed liquid and solid growth medium to produce the bacteriocin in the liquid growth medium; and
    (b) removing impurities present in the liquid by ultrafiltration to produce a retentate containing the bacteriocin, wherein the retentate has a higher activity per unit volume than the liquid growth medium containing the bacteriocin in the liquid, and wherein the bacteriocin has a molecular weight between about 4,000 and 5,000 daltons, is stable in boiling water and is tasteless in foods at levels which inhibit the growth of bacteria present in the food.

11. The bacteriocin of Claim 10 wherein the bacteriocin is in the form of a dry powder.

12. The bacteriocin of Claim 10 which is effective in an amount up to 10 percent by weight of the food without imparting a taste to the food.

13. The bacteriocin of Claim 10 wherein in the method the ultrafiltration separates the bacteriocin from the liquid at a molecular weight cutoff for the ultrafiltration of between 4,000 and 16,000 daltons to provide the retentate containing the bacteriocin.

14. The bacteriocin of Claim 10 wherein in the method the growth medium includes a protein hydrolysate, an amino acid, a sugar and mineral supplements which stimulate the production of the bacteriocin.

15. The bacteriocin of Claim 10 wherein in the method the liquid of the retentate is treated with a precipitation agent to precipitate the bacteriocin which is separated from the liquid.

16. The bacteriocin of Claim 15 wherein the precipitation agent is ammonium sulfate.

17. The bacteriocin of Claim 15 wherein in the method the precipitated bacteriocin is lyophilized to a dry powder.

18. The bacteriocin of Claim 10 with an AU per ml of at least about 100 AU/ml when assayed against an indicator strain of Pediococcus pentosaceus grown on an agar plate.

19. The bacteriocin of Claim 10 wherein in the method the liquid of the retentate is treated with a precipitating agent to precipitate the bacteriocin and then separated from the liquid and then the bacteriocin is resuspended in a buffer and dialyzed to remove impurities in the bacteriocin.

20. The bacteriocin of Claim 19 wherein the buffer includes ammonium bicarbonate.

**21.** The method of Claim 7 wherein the buffer includes ammonium bicarbonate.

**22.** The bacteriocin of Claim 10 produced by a Pediococcus acidilactici.

**23.** The bacteriocin of Claim 10 produced by Pediococcus acidilactici NRRL-B-18050.